(19) **Europäisches Patentamt**
**European Patent Office**
**Office européen des brevets**

(11) **EP 3 085 414 B1**

(12) **EUROPEAN PATENT SPECIFICATION**

(45) Date of publication and mention
of the grant of the patent:
**18.03.2020 Bulletin 2020/12**

(51) Int Cl.:
***A61N 1/36*** (2006.01)

(21) Application number: **16164037.0**

(22) Date of filing: **06.04.2016**

(54) **DEVICE FOR SELECTIVE NERVE STIMULATION**

VORRICHTUNG ZUR SELEKTIVEN NERVENSTIMULATION

DISPOSITIF DE STIMULATION NERVEUSE SÉLECTIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2015 US 201562150865 P**

(43) Date of publication of application:
**26.10.2016 Bulletin 2016/43**

(73) Proprietor: **BIOTRONIK SE & Co. KG**
**12359 Berlin (DE)**

(72) Inventors:
• **Baru, Marcelo**
**Tualatin, OR 97062 (US)**

• **Kibler, Andrew B.**
**Lake Oswego, OR 97035 (US)**

(74) Representative: **Galander, Marcus**
**Biotronik Corporate Services SE**
**Corporate Intellectual Property**
**Sieversufer 7 - 9**
**12359 Berlin (DE)**

(56) References cited:
**EP-A1- 1 392 393      WO-A2-2007/012907**
**US-A1- 2003 212 440   US-A1- 2008 300 657**
**US-A1- 2010 191 311   US-A1- 2014 046 407**

**Description**

The invention relates to an implantable pulse generator (IPG) that is connected or can be connected to a stimulation lead having a plurality of stimulation electrodes for delivery of stimulation pulses and that comprises at least one stimulation unit and control unit configured to generate electric stimulation pulses for nerve stimulation, e.g. vagus nerve stimulation (VNS). The invention further related to a method for nerve stimulation, e.g. vagus nerve stimulation (VNS).

Vagus nerve stimulation (VNS) recently emerged as a potential progression-preventing and treatment option for congestive heart failure (CHF) patients. Experimental data have demonstrated that stimulation of the vagus nerve at the cervical level is able to reverse ventricular remodeling of the failing heart. There is also evidence that increasing parasympathetic activity may stimulate the production of nitric oxide, and reduce the devastating inflammatory process involved in heart failure. Present VNS apparatuses for CHF involve an implanted nerve cuff electrode that connects via wires to an IPG in the patient's chest. A standard pacemaker sensing lead in the ventricle has been proposed in prior art for the purpose of cardiac sensing for synchronous delivery of VNS pulses in the cardiac refractory period, although other prior art apparatuses operate asynchronously to the cardiac cycle. Stimulation of both the right and left vagus nerves are disclosed in prior art for CHF treatment.

Selective stimulation of a vagus nerve region with a majority of parasympathetic cardiac fibers for the purpose of CHF treatment, while minimizing the possible stimulation of large-diameter fibers that innervate the pharynx and the larynx, is desired to reduce side effects.

US 5,199,430 A discloses the utilization of a nerve cuff electrode and quasi-trapezoidal (QT) pulses to selectively initiate action potentials adjacent to a central electrode and to block the propagation of action potentials adjacent to the end electrodes along the larger-diameter nerve fibers but not the smaller-diameter nerve fibers.

US 7,389,145 B2 discloses a specific electrical stimulus waveform that can be applied to cause blocking of nerve activity. It consists of a first sub-threshold cathodic phase immediately followed by an anodic phase, i.e. when the cathodic current reaches zero the pulse is reverted with a non-zero amplitude. This biphasic pulse is repeated continuously and the amplitude may be increased to block other smaller-diameter fibers as desired.

US 2010/0191311 A1 discloses the utilization of a nerve cuff electrode and two stimulation trains: a low-frequency train and a high-frequency one, delivered either using the same electrodes or different electrodes. In accordance with an embodiment of this prior art, the low frequency train recruits the desired cardiac fibers for treatment while the activity of certain nerve branches, as e.g. those innervating the larynx or pharynx, are blocked via the high-frequency train. An identical technique, showing reverse nerve fiber recruitment, was disclosed by Baratta et al. in 1989 although not in the vagus nerve [Baratta et al. "Orderly Stimulation of Skeletal Muscle Motor Units with Tripolar Nerve Cuff Electrode", IEEE Transactions on Biomedical Engineering, vol. 39, no. 8, pp. 836 - 843, August 1989].

US 8,615,294 B2 discloses a nerve cuff with ring electrodes. Ring electrodes have been used in nerve cuff electrodes since the early 70s; it is not something new disclosed by this prior art.

It is an object of the invention to provide means for improved nerve stimulation, in particular an improved implantable pulse generator (IPG) and an improved method for vagus nerve stimulation (VNS).

According to the invention, this object is achieved by an IPG that is connected or can be connected to a stimulation lead having a plurality of stimulation electrodes for delivery of stimulation pulses. The IPG comprises at least one stimulation unit that is configured to generate electric stimulation pulses for nerve stimulation. The IPG further comprises a control unit that is configured to trigger delivery of generated electric stimulation pulses via selected electrodes of the plurality of stimulation electrodes wherein said electric stimulation pulses form a pulse train comprising:

i) an initial selective-arrest phase for the large-diameter fibers in the vicinity of the selected electrodes;

ii) followed by a phase where a charge-balanced Alternating Current (AC) is applied between the same or other selected electrodes;

iii) and where such AC is briefly unbalanced to effectively deliver nerve stimulation therapy pulses, returning to charge-balanced operation in between therapy pulses.

In an embodiment of the invention, the control unit is further configured to select one or more electrodes of the plurality of stimulation electrodes.

Preferably, the plurality of stimulation electrodes comprises at least one ring electrode and a plurality of contacts that are electrodes with a smaller contact surface than said ring electrode.

In another embodiment of the invention, the plurality of stimulation electrodes comprises at least two ring electrodes that are axially spaced from each other and wherein said contacts are arranged between said at least two ring electrodes.

Preferably, the control unit of the implantable pulse generator is configured to trigger a pulse train for nerve stimulation therapy that is time duty-cycled.

In a preferred embodiment of the invention, the control unit uf the implantable pulse generator is configured to trigger bipolar stimulation between two of the contacts, or a contact and at least one ring.

Preferably, the control unit of the implantable pulse generator is configured to terminate nerve stimulation therapy by interruption of the pulse train.

In another preferred embodiment of the invention, the control unit of the implantable pulse generator is configured to trigger delivery of a pulse train effecting a passive charge-balancing phase involving short circuiting of the selected active contact(s) and ring(s) as required during the pulse train and immediately after termination of the nerve stimulation therapy. The charge-balancing period may involve two stages with different contact(s) (102) and ring(s) (104).

According to another aspect of the invention, at least one stimulation unit of the implantable pulse generator is configured to generate a kHz alternating current (AC), wherein said at least one stimulation unit comprises or is connected to a low-Q Class-E switched amplifier for generating said kHz alternating current (AC).

Preferably, the implantable pulse generator is configured to effect the selective-arrest phase by rectification of the kHz alternating current (AC) with a suitable envelope, in particular an envelope approximating a quasi-trapezoidal (QT) pulse.

In a preferred embodiment of the invention, the implantable pulse generator is configured to effect said selective-arrest phase by means of pre-depolarization sub-threshold pulses.

Further, but not forming part of the invention, a method for selective neural stimulation and in particular for vagus nerve stimulation (VNS) is described, said method comprising the steps of providing a stimulation pulse train, said train comprising stimulation pulses that are configured to effect:

  i) an initial selective-arrest phase for the large-diameter fibers in the vicinity of selected electrodes;

  ii) followed by a phase where a charge-balanced Alternating Current (AC) is applied between the same or other selected electrodes;

  iii) and where such AC is briefly unbalanced to effectively deliver nerve stimulation therapy pulses, returning to charge-balanced operation in between therapy pulses.

In a preferred embodiment of the invention, the initial selective-arrest phase for the large-diameter fibers is achieved by hyperpolarization of such fibers. In a following phase, selective arrest is achieved by sub-threshold membrane depolarization, causing inactivation of sodium channels while avoiding action potential generation. The transition from hyperpolarization to high-frequency induced sub-threshold depolarization is an important aspect of the present invention, as it enables conduction block while not producing an initial supra-threshold depolarization at the onset of blocking stimulation.

According to one aspect of the invention, the nerve stimulation therapy is delivered to the vagus nerve, wherein the initial selective-arrest phase for the large-diameter fibers is delivered to nerves fibers that innervate the pharynx and the larynx.

Accordingly, an implantable apparatus and method for cervical vagus nerve stimulation (VNS) is provided, with the capability of selectively stimulating regions of a vagus nerve with a majority of parasympathetic cardiac fibers while minimizing the possible stimulation of the large-diameter fibers that innervate the pharynx and the larynx (should they be present). This implantable apparatus and method may be particularly suitable to the management of congestive heart failure (CHF). The implantable pulse generator (IPG) preferably comprises or is connected to a multi-contact nerve cuff electrode. In a preferred embodiment of the invention, the stimulation lead is a multi-contact nerve electrode. The IPG is configured to be implanted in the patient's chest. The nerve cuff electrode may have at least three contacts, circumferentially distributed at equal increments and located towards the center of the cuff, and at least one ring electrode near its edge.

The invention involves the recognition that cervical vagus nerve stimulation (VNS) by means of an implanted cuff electrode may suffer from unwanted recruitment of large-diameter fibers which may translate into undesired side effects such as voice hoarsening, coughing, shortness of breath, and pain in the neck area during therapy delivery for cardiovascular effects. The present invention provides a solution which may limit the recruitment of large-diameter fibers to a single event, at the beginning of each VNS therapy pulse train, thus minimizing such unwanted side effects.

Nerve stimulation therapy, in particular VNS, may be time duty-cycled and preferably delivered by bipolar stimulation between two of the contacts, or a contact and at least one ring. Given the limited nerve cuff length available for cervical implantation, undesired recruitment of large-diameter fibers (during VNS therapy) may be minimized by a pulse train composed of:

i) an initial selective-arrest phase for the large-diameter fibers caused by stimulation applied between a selected contact and ring(s);

ii) followed by a phase where a charge-balanced alternating current (AC) is applied between a selected contact, and ring(s) or alternatively another contact in which case ring(s) is(are) disconnected;

iii) and where such AC is briefly unbalanced to effectively deliver VNS therapy pulses, returning to charge-balanced operation in between therapy pulses.

Accordingly, laryngeal and pharyngeal nerve fibers (large-diameter) are arrested by a first phase of the stimulation train for arresting nerve activity, e.g. by using a stimulation signal with quasi-trapezoidal (QT) form, which can be generated by the envelope of the rectified AC signal. Arresting activity by hyperpolarization of large-diameter fibers prevents over muscle response which would otherwise be evoked by the stimulation for blocking such fibers using high frequency in the next step. Then, stimulation using charge-balanced, high frequency (HF, preferably kH range) for blocking large-diameter fibers activity is applied, wherein the HF signal is modulated in a way that both VNS for cardiovascular effects and blocking of the laryngeal and pharyngeal nerve fibers are ensured (e.g. unbalance of HF signal generates VNS).

Therapy may be terminated by interruption of the pulse train. A passive charge-balancing phase, for neutrality purposes may follow, involving short circuiting of the selected active contact(s) and ring(s). A similar balancing phase may be required during the pulse train delivery. This charge-balancing phase may involve two stages with different contact(s) and ring(s).

In a preferred embodiment of the present invention, a single, multi-phase waveform pulse train is utilized comprised of an initial phase with a net charge component to arrest action potentials of large-diameter fibers, which transitions into a continuous, charge-balanced AC waveform (preferably 32,768 Hz or submultiple down to hundreds of Hz) to prevent such fibers from conducting after the conclusion of the selective-arrest phase. Temporary unbalancing of the AC waveform, or injection of larger cathodic pulses during a short quiescent period of the waveform, is utilized to induce a net cathodic impulse and recruit smaller-diameter, unblocked cardiac fibers for therapy. Once block is established, the AC waveform may be switched off (e.g. < 10 ms) without affecting the blocking effect.

In a preferred embodiment, a bipolar cuff arrangement, with a central cathode (a contact) flanked by an edge ring connected as anode (preferably the one proximal to the vagus heart innervation), implement the required configuration for the selective-arrest phase of the large-diameter fibers. A suitable "pseudo" pulse is utilized during this phase which may be implemented by injecting a rectified version of the AC waveform with an envelope approximating a desired continuous equivalent pulse. Cardiac fibers may be stimulated during this selective-arrest phase. Following its termination, the cuff configuration may automatically be switched to bipolar stimulation between the cathode contact and another contact selected to work as anode, in which case the ring(s) is(are) disconnected, or continue with the contact-ring(s), and the stimulation transitioned into a continuous, charge-balanced AC waveform with an amplitude that prevents the large-diameter fibers from firing action potentials. To deliver therapy pulses, i.e. recruit cardiac fibers, the AC waveform may be temporarily unbalanced (e.g. rectified for tens to hundreds of $\mu$s) and returned to charge-balanced operation at the end of a therapy pulse. VNS therapy can be terminated without triggering a large-diameter fiber action potential. A passive charge-balancing period (ms to tens of ms range), utilizing the active contact(s) and ring(s) involved, may be performed during the AC-waveform quiescent period, and immediately after pulse train termination. Such charge-balancing phase may be done in two different stages involving different contact(s) and ring(s).

In a preferred embodiment, kHz alternating current (AC) is utilized and generated by a switched amplifier, in particular a low-Q Class-E amplifier where efficiency is traded for harmonic distortion.

Given that linearity is not required for kHz AC nerve stimulation, a Class-E amplifier is preferred for the implementation of the stimulation circuitry. To reduce the number of components, a single inductor, single capacitor Class-E amplifier is proposed with DC blocking capacitors in series with each contact and ring. Analog switches allow connecting/disconnecting the different contacts and ring(s) and implementing half-way rectification via some switches parasitic diodes. The Class-E amplifier may be powered from battery voltage, voltages generated from it, or other regulated voltages when alternative powering is utilized in the implantable pulse generator (IPG).

The selective-arrest phase may be implemented by rectification of the kHz alternating current (AC) with a suitable envelope, e.g. an envelope approximating a quasi-trapezoidal (QT) pulse (hereinafter referred as pseudo QT). In another preferred embodiment, pre-depolarization sub-threshold pulses are instead utilized to implement the selective-arrest phase.

In an alternative embodiment, an H-bridge with an arbitrary waveform generator is instead utilized to implement the pulse train.

The automatic selection of the best contact(s) or contact-ring(s) for therapy, as well as other aspects of closed-loop operation such as intrathoracic far-field electrogram (ff-EGM) recording and processing, and communication with an external programmer or bedside patient messenger, are further aspects that may complement the present invention.

As mentioned before, the present invention provides a solution which may limit the recruitment of large-diameter fibers to a single event, or no action potential event, at the beginning of each VNS therapy pulse train, thus minimizing such unwanted side effects.

The above and other aspects, features and advantages of the present invention will be more apparent from the following more particular description thereof, presented in conjunction with the following drawings wherein:

Fig. 1      shows a preferred embodiment of the present invention

Fig. 2      is a schematic diagram of some components of an implantable pulse generator (IPG)

Fig. 3      conceptually describes the connection for implementing the selective-arrest phase when vagus nerve stimulation (VNS) therapy is to be delivered

Fig. 4      conceptually describes the connection after the selective-arrest phase is completed.

Fig. 5      is a schematic representation of the stimulation circuitry for the purpose of delivering VNS therapy.

Fig. 6      illustrates an extension of the circuit in Fig. 5 for operation with multiple electrodes.

Fig. 7      shows a circuit connection for delivering a selective-arrest phase using a pseudo quasi-trapezoidal (QT) pulse.

Fig. 8      illustrates the shape of the current flowing through tissue exiting stimulation contact.

Fig. 9      illustrates a waveform that occurs when the rectifying characteristic of the current flowing through tissue disappears and transits to a steady-state, charge-balanced alternating current (AC) waveform.

Fig. 10      schematically shows a typical VNS therapy train.

Fig. 11      shows a circuit connection for achieving charge-balancing.

Fig. 12      shows an embodiment wherein analog switches are implemented with back-to-back PMOS transistors.

Fig. 13      shows an embodiment wherein analog switches are implemented using back-to-back NMOS transistors.

Fig. 14      shows an alternative embodiment wherein the therapy train is delivered by an H-bridge circuit.

Fig. 15      shows an example therapy train (not to scale) with a selective-arrest phase.

Fig. 16      shows a common model of the sodium channel gate response at a range of transmembrane potentials.

The following description is of the best mode presently contemplated for carrying out the invention. This description is not to be taken in a limiting sense, but is made merely for the purpose of describing the general principles of the invention. The scope of the invention should be determined with reference to the claims.

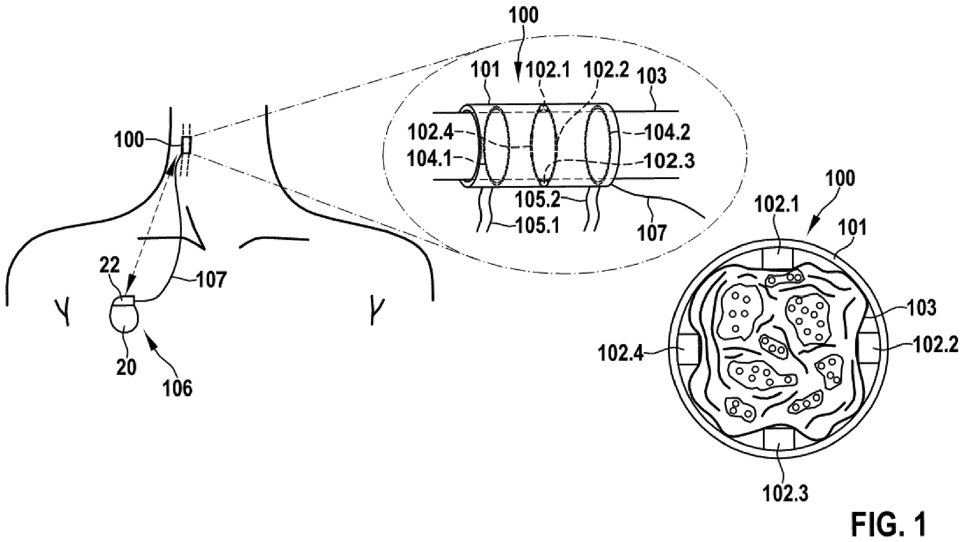

Figure 1 shows a preferred embodiment of the present invention. Basic components of this embodiment are nerve cuff electrode 100, an implantable pulse generator (IPG) 106 and implantable electric conduit (e.g. isolated wires) 107.

According to this preferred embodiment, the nerve cuff electrode 100 may be constructed using a silicone rubber tube 101, with centered exposed Pt/Ir contacts 102 (of 2 mm$^2$ each) in its interior wall distributed circumferentially, and at least a ring 104.1 towards the edge proximal to the vagus heart innervation, all in contact with the right vagus nerve 103 surface. Contacts 102, and rings 104.1 and 104.2 (a second ring may be placed on the distal edge), are stimulation electrodes.

The cuff 100 may be self-coiling or it may include other closing mechanisms such as a piano hinge with a nylon suture (not shown). Biocompatible strings 105 may be built on the cuff 100 outer wall to open it for easy implantation around the nerve 103. In a second preferred embodiment, the contacts 102 are made of fractal Ir for higher charge-injection capacity. In an alternative embodiment, ring(s) 104 may be formed out of individual segmented electrodes in a circumferential arrangement so that when they are driven in synchrony, the electrical field effectively matches that formed by a complete ring electrode.

The cuff 100 is connected to IPG 106, located in the patient's chest area, via a subcutaneously-implanted isolated multi-wire 107 which provides electrical connection to the contacts 102 and ring(s) 104.

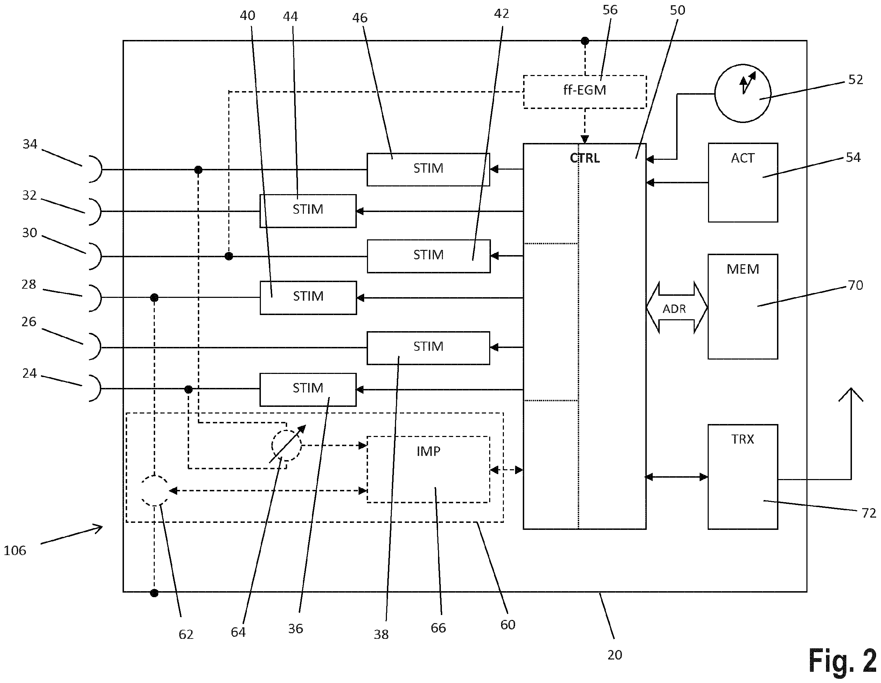

Figure 2 is a schematic diagram of some components of an implantable pulse generator (IPG) 106.

As can be taken from Fig. 2, the IPG 106 comprises a case (IPG case) 20 and a header 22 (see Fig. 1) for connection of electric conduit107. Header 22 comprises a number of connectors 24, 26, 28, 30, 32 and 34 (at least five) that can electrically connect to connectors of the electrical conduit 107. Thus, an electric connection between connectors 24, 26, 28, 30, 32 and 34 and electrodes (contacts and rings) 102 and 104 respectively, of nerve cuff electrode 100, can be made.

Within IPG case 20 one or more stimulation units 36, 38, 40, 42, 44 and 46 are arranged that are electrically connected to connectors 24, 26, 28, 30, 32 and 34, respectively, and configured to generate stimulation pulses and to deliver such stimulation pulses via a respective connector 24, 26, 28, 30, 32 and 34. It should be noted that instead of one stimulation unit for each connector and thus for each electrode 102 and 104, a single stimulation unit and a switch matrix can be provided. In the latter embodiment, delivery of stimulation pulses via selective connectors and thus via selected electrodes 102 and 104 can be achieved by the switch matrix. In another embodiment, all contacts 102 are switched in parallel to each other and thus only one connector and one stimulation unit is needed. In the latter embodiment, no selection of contacts 102 for delivery of stimulation pulses is possible.

In the embodiment of Fig. 2, each stimulation unit 36, 38, 40, 42, 44 and 46 is connected to and controlled by a control unit 50. Control unit 50 controls generation and triggers delivery of stimulation pulses by stimulation units 36, 38, 40, 42, 44 and 46.

The stimulation pulses to be generated and triggered by each stimulation unit 36, 38, 40, 42, 44 and 46 are tailored for vagus nerve stimulation (VNS).

Control unit 50 further is connected to a time signal generator 52 that supplies a time base to control unit 50.

Further, an activity sensing unit 54 may be provided for sensing movements of the patient via movements of the IPG 106, preferably in three spatial dimensions. This activity sensor would deliver the activity signal to control unit 50.

Control unit 50 further may be connected to a far-field electrogram (ff-EGM) sensing unit 56 that is configured to generate a ff-EGM signal representing a far-field electrogram. In order to record such ff-EGM signal, far-field sensing unit 56 is connected to at least one of connectors 24 to 34 and thus to one of the electrodes 102 or 104 of nerve cuff electrode 100. Another input of a ff-EGM sensing unit 56 is connected to IPG case 20. Thus, ff-EGM sensing unit 56 can sense voltages between at least an electrode 102 or 104 and IPG case 20 that result from electric potentials which are caused by the activity of a patient's heart. Far-field electrogram sensing unit 56 is configured to supply a ff-EGM signal to control unit 50. The ff-EGM signal represents the heart activity of a patient. From the ff-EGM signal inter alia a heart rate of a patient can be determined.

Control unit 50 may further be connected to an impedance measuring unit 60 that comprises a constant current source 62 for generating and delivering biphasic impedance measuring pulses. Current source 62 may electrically connect to IPG case 20 and to at least one of connectors 24 to 34 and thus to at least one of the electrodes 102 or 104 of nerve cuff electrode 100. Impedance measurement unit 60 further comprises a voltage sensing unit 64 that is configured to measure a voltage difference between at least one electrode 102, 104 of nerve cuff electrode 100 and the IPG case 20, or between at least two electrodes 102, 104, in response to delivery of current pulses by the current source 62. Current source 62 and voltage sensing unit 64 are connected to an impedance determination unit 66 of the impedance measurement unit 60. Impedance determination unit 66 is configured to generate an impedance signal depending on the voltages measured by voltage sensing unit 64 and to supply such impedance signal to control unit 50. The impedance signal generated by impedance measurement unit 60 allows assessing the status of electrodes 102, 104.

Control unit 50 further may be connected to a memory unit 70 that may serve to store signals recorded by control unit 50 or programs that control the operation of control unit 50.

In order to wirelessly communicate recorded signals to an external device or to receive program instructions, a telemetry unit 72 is provided that is also connected to control unit 50.

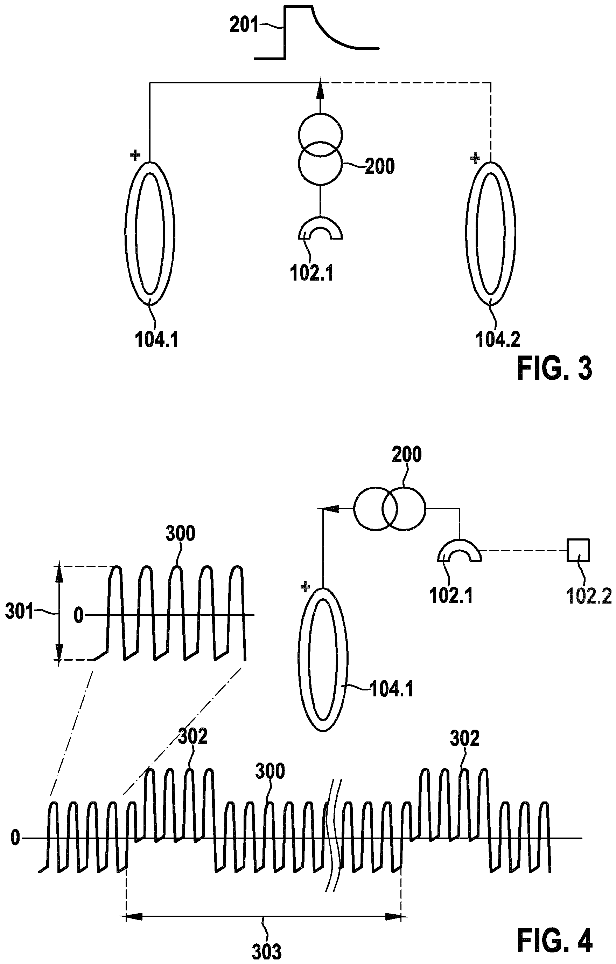

Figure 3 conceptually describes the preferred connection for implementing the selective-arrest phase when VNS therapy is to be delivered. Without losing generality, let us say contact 102.1 has been determined as the preferred cathode and ring 104.1 as anode. In such case, the control unit 50 (and associated stimulation unit) injects an electrical pulse 200 (preferably current-based) that exits contact 102.1. The electrical pulse 200 may have, for example, a quasi-trapezoidal (QT) envelope 201, i.e. a square leading edge, a plateau pulse width (typically 50 to 500 $\mu s$), followed by an exponential trailing phase with a fall (90% to 10%) of similar duration to the plateau pulse width. In a second preferred embodiment, both rings 104 are utilized as anodes for therapy. In a third preferred embodiment, the nerve cuff electrode 100 may only include ring 104.1 and the contacts 102 may be off-centered.

Once the selective-arrest phase is completed, the configuration of Fig. 3 may be automatically switched by the control unit 50 to the one shown in Fig. 4 (dashed line), assuming without losing generality that VNS therapy is preferably to be delivered between contacts 102.1 (cathode) and 102.2 (anode), or continue with the ring 104.1 as anode. Regardless of this post configuration, the control unit 50 will continue outputting electrical pulses 200 between an anode and the selected cathode contact 102.1. Let's describe the case where ring 104.1 remains to be the anode. The current 200 may now transition to a charge-balanced, non-pure sinusoidal waveform 300 of sufficient amplitude 301 (later referred to as baseline) to prevent large-diameter fibers from firing action potentials. To deliver VNS therapy, the sinusoidal waveform 300 is briefly unbalanced (e.g. rectified) creating the pseudo pulses 302 and brought back to baseline in between. This envelope change may have different shapes for stimulation and may generally be repeated with a period 303 typically between 10 to 100 ms during therapy delivery.

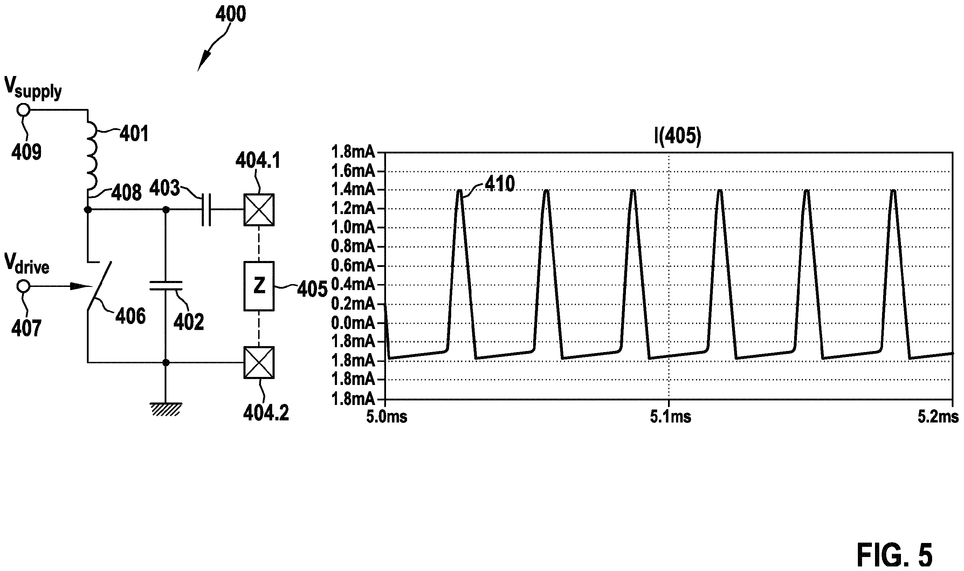

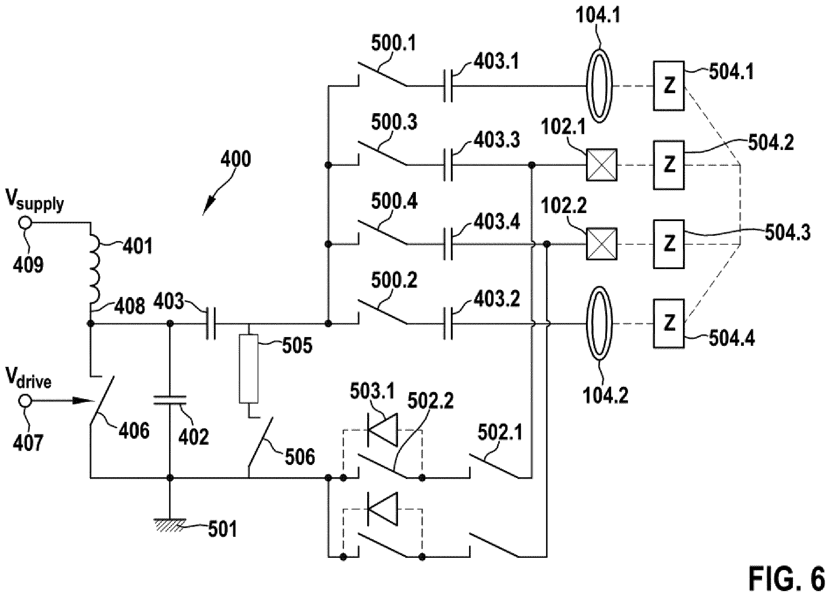

Figure 5 is a schematic representation of the preferred stimulation circuitry for the purpose of delivering VNS therapy. This circuit 400 is a switched-mode Class-E amplifier with a parallel LC circuit composed of 401, 402. A DC blocking capacitor 403 may be placed in series with the electrodes 404 in contact with tissue (the impedance between them is represented by Z 405). Only two electrodes 404 are shown to describe the circuit, which may be any contact 102 or ring 104. Operation with multiple electrodes 404 is presented in Fig. 6 and described later on.

Capacitor C 402 includes the parasitic capacitance of the analog switch 406. Such switch 406 is driven by $V_{drive}$ 407 in such a way as to provide switching between its on-state and off-state operation modes. As a result, the voltage in node 408 is determined by the transient response of the LC load network (401, 402) when the switch 406 is off. For optimum operation $V_{drive}$ 407 may have a 50% duty cycle. This circuit 400 is powered by $V_{supply}$ 409.

Assuming the analog switch 406 is ideal, i.e. has zero saturation voltage, zero saturation resistance, infinite off-state resistance, and its switching action is instantaneous and lossless, and that capacitor C 402 is independent of node voltage 408 and assumed linear, the optimum values for L 401 and C 402 can be derived from:

$$L = 0.41 \frac{Re(Z)}{w} \qquad C = \frac{1.025}{w \, Re(Z)}$$

where Re(Z) is the resistive part of the electrodes-tissue impedance 405 and w is the angular frequency of $V_{drive}$ 407.

Since $V_{drive}$ 407 may preferably be in the kHz range, Z 405 may be primarily resistive. At 32,768 Hz for example, Re(Z) may be in the order of 1,000 $\Omega$, and the equations above determine a value of approximately 2 mHy and 5 nF for L 401 and C 402 respectively. Analog switch 406 may be an NMOS transistor. The circuit 400 generates a current through Z 405 with shape 410 when $V_{supply}$ 409 is 1.2 V.

With the addition of extra analog switches, the circuit of Fig. 5 may be expanded as shown in Fig. 6 to implement a preferred pulse train for VNS therapy. The rings 104.1 and 104.2 are in series with DC blocking capacitors 403.1 and 403.2, and may connect to node 408 via analog switches 500.1 and 500.2 respectively. Contacts 102.1 and 102.2, being considered for circuit examples, may also connect to node 408 through similar analog switches 500.3 and 500.4 and DC blocking capacitors 403.3 and 403.4 respectively. Contact 102.1 may also connect to the circuit ground 501 via analog switches 502.1 and 502.2. Parasitic diode 503.1, of analog switch 502.2, is highlighted as it is utilized in the implementation of the selective-arrest phase of the pulse train in a preferred embodiment as explained below. Similar components (not all of them shown) connect the remaining contacts. Electrode-tissue impedances from rings 104.1, 104.2, and contacts 102.1, 102.2, are represented by Z blocks 504.1 - 504.4. Finally, resistor 505 and analog switch 506 are utilized for charge-balancing as it will also be described later on.

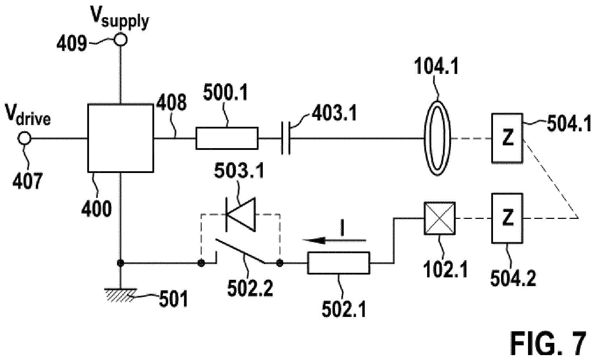

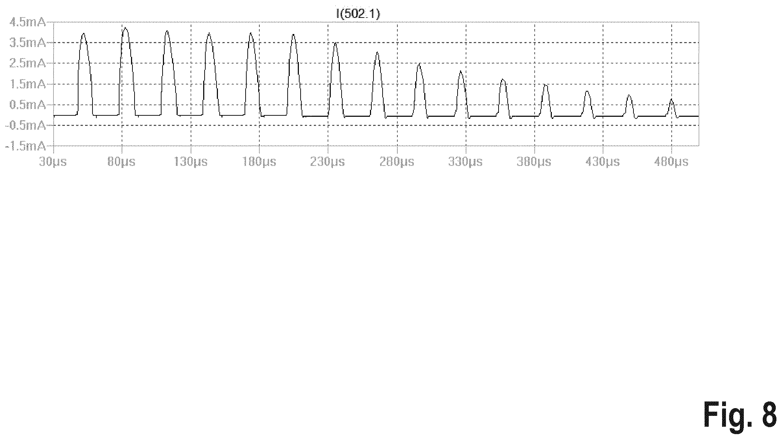

Figure 7 shows the preferred circuit connection of Fig. 6, in the example being described, for delivering a selective-arrest phase using a pseudo quasi-trapezoidal (QT) pulse. In this configuration, analog switch 500.1 is closed which connects ring 104.1 to node 408 through DC blocking capacitor 403.1. On the other hand, analog switch 502.1 is closed connecting contact 102.1 to circuit ground 501 through the parasitic diode 503.1 of analog switch 502.2 (which remains open). The switched-mode Class-E amplifier circuit 400 is connected to node 408 and circuit ground 501. When $V_{drive}$ 407 drives analog switch 406 (inside 400, not shown) and $V_{supply}$ 409 is ramped up linearly for 200 $\mu$s from 1.2 V to a final value of 2.0 V, and linearly ramped down reaching 1.2V at 500 $\mu$s, the current flowing through tissue exiting contact 102.1 I (502.1) has the shape shown in Fig. 8.

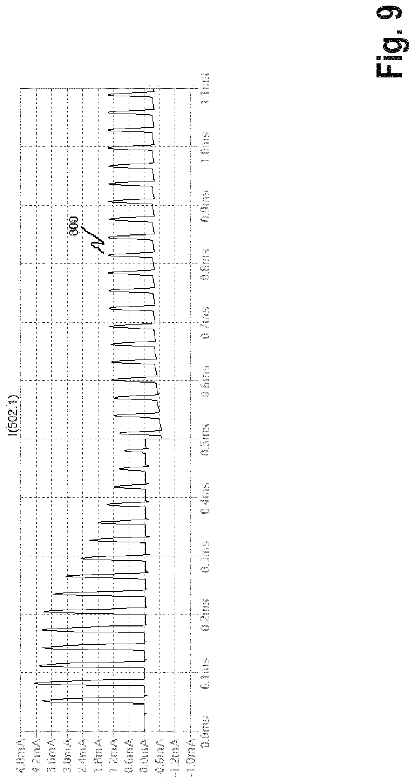

At the end of the selective-arrest period of Fig. 8, switch 502.2 is closed. This implies the rectifying characteristic of the current I (502.1) flowing through tissue will disappear and transition (after approximately 150 $\mu$s in the example selected) to the steady-state, charge-balanced alternating current (AC) waveform 800 shown in Fig. 9. The amplitude of such waveform 800 prevents the largest diameter fibers from conducting. $V_{drive}$ 407 is uninterrupted and $V_{supply}$ 409 maintained at 1.2 V. Once block is established, the waveform 800 may be switched on and off as temporary interruptions will not affect the blocking effect on the large-diameter fibers. During the off time, passive charge-balancing may be performed for neutrality purposes as desired.

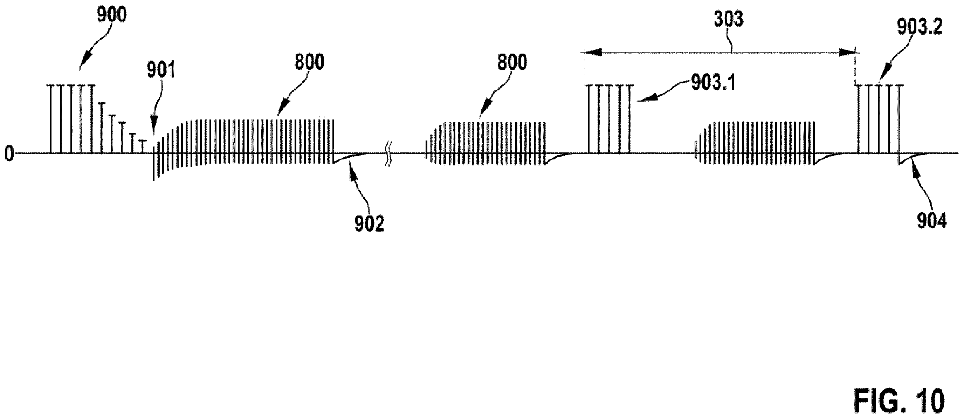

To deliver therapy, switch 502.2 may be opened to create a pseudo pulse similar to the creation of the selective-arrest phase. Figure 10 schematically shows a typical therapy train that starts with the selective-arrest phase 900 and transitions (region 901) to a steady-state, charge-balanced alternating current (AC) waveform 800. Once block of the large-diameter fibers is achieved, the AC waveform 800 may be turned off and the charge accumulated in the DC blocking capacitors and electrode-tissue capacitances bled off via a passive charge-balancing phase 902. The AC waveform 800 may be turned on and off while maintaining the blocking effect. Therapy can be delivered by unbalancing the AC waveform 800. It may be on or off when a therapy pulse 903 needs to be delivered. Therapy pulses may have different envelopes with respect to the selective-arrest phase 900. In Fig. 9 for example these pulses 903 have a rectangular envelope. At the end of the VNS therapy pulses, a global balancing phase 904 takes place for neutrality purposes.

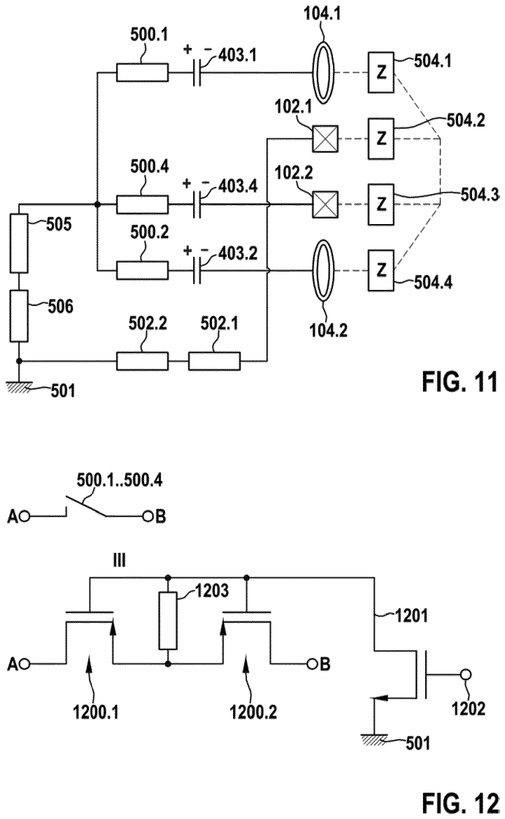

To initiate a passive charge-balancing phase 902, 904, the active analog switches that connect the participating contact(s) 102 and ring(s) 104 are opened, $V_{supply}$ 409 and $V_{drive}$ 407 are brought to circuit ground voltage 501, and analog switch 506 (see Fig. 6) is closed. This will dissipate the energy left in L 401 and C 411 through resistor 505. Charge-balancing may then be achieved with the circuit connection of Fig. 11. Analog switches 500.1, 500.2, 502.1, 502.2, 506, and 500.4 (assuming ring 104.2 and contact 102.2 participated in the therapy) are closed discharging the voltage accumulated in the DC blocking capacitors 403.1, 403.2 and 403.4, and in the electrode-tissue capacitance of 504.1, 504.2, 504.3 and 504.4, through resistor 505 (e.g. kΩ range). This charge-balancing period may be implemented in two stages, i.e. one corresponding to the selective-arrest phase where in the example only analog switches 500.1, 500.2, 502.1, 502.2 and 506 are closed for a finite period of time (ms to tens of ms range) and another one where analog switches 500.1 and 502.2 are opened and 500.4 closed instead for another finite period of time if contact 102.2 was utilized as anode for therapy.

Analog switches 500.1..500.4 (and the equivalents not drawn for the other contacts) may be implemented with back-to-back PMOS transistors 1200.1 and 1200.2 as shown in Fig. 12, enabled by NMOS transistor 1201 whose gate 1202 may vary between ground voltage 501 (switch off) and a positive voltage (switch on). The turn-off of transistors 1200.1 and 1200.2 may be passively done by resistor 1203 (e.g. 100 kΩ).

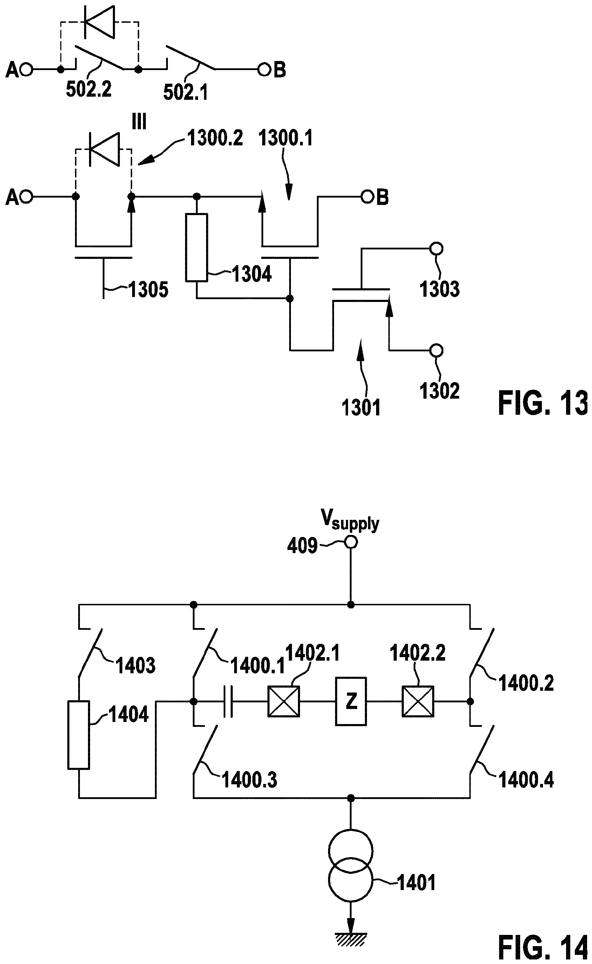

Analog switches 502.1 and 502.2 (and the equivalents not drawn for the other contacts) may be implemented using back-to-back NMOS transistors 1300.1 and 1300.2 as shown in Fig. 13, with transistor 1300.1 enabled by PMOS transistor 1301 with its source 1302 connected to a positive voltage and its gate 1303 varying between such positive voltage (switch off) and ground voltage 501 (switch on). The turn-off of transistor 1300.1 may be passively done by resistor 1304 (e.g. 100 kΩ). The gate 1305 of transistor 1300.2 is driven by the control logic 50 in the IPG 106 to implement the VNS therapy.

In an alternative embodiment, the therapy train is delivered by an H-bridge circuit as schematically shown in Fig. 14. Analog switches 1400.1 and 1400.4 allow the stimulating current 1401 to flow from electrode 1402.1 to 1402.2, whereas analog switches 1400.2 and 1400.3 allow current 1401 to flow in the opposite direction. The stimulation current 1401 can be re-programmed on the fly by the control logic 50 in the IPG 106 to implement arbitrary shapes.

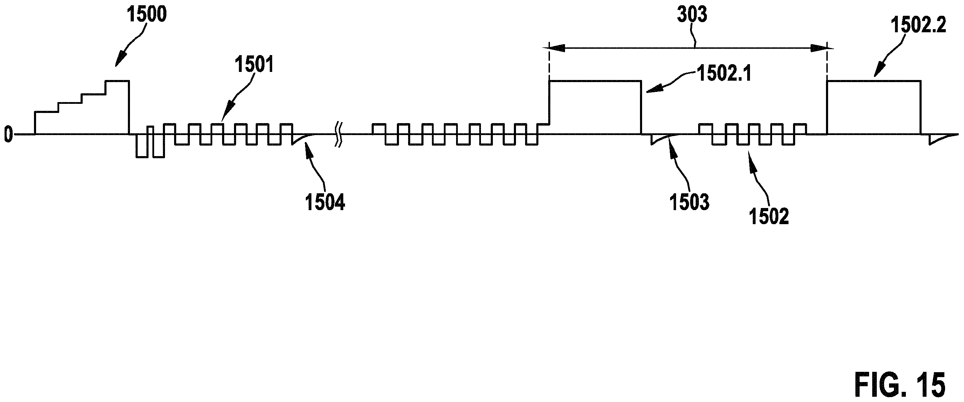

Figure 15 shows an example therapy train (not to scale) with a selective-arrest phase 1500 implemented using sub-threshold pre-depolarization pulses that transitions into a charge-balanced rectangular waveform 1501 of sufficient amplitude to keep the largest diameter fibers from conducting. Therapy pulses can be square pulses 1502, with associated passive charge-balancing 1503, created by unbalancing the waveform 1501 and increasing their amplitude. The passive charge-balancing phase 1503, as well as passive charge-balancing phases 1504 when waveforms 1501 are temporarily stopped, may be implemented with analog switch 1403 and current-limiting resistor 1404 of Fig. 14.

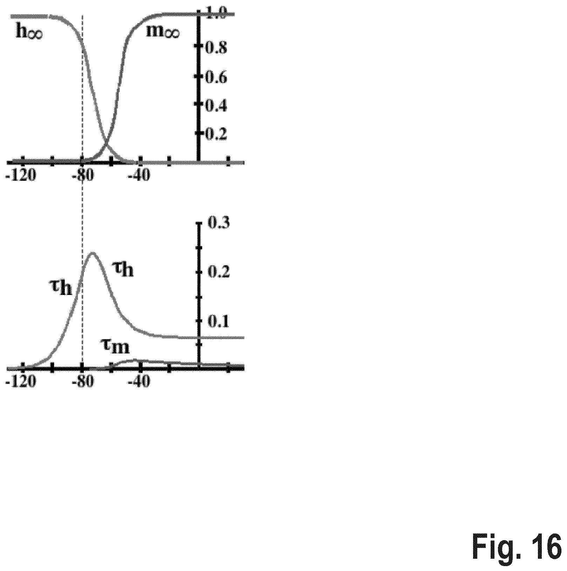

Figure 16 shows the voltage and time response of a well-known model of the sodium channel, primarily responsible for depolarization in neural excitation. At low transmembrane potentials, for example -120mV, the m-gate responsible for allowing sodium ion transport has a probability of being open of near 0, indicating that the channel is closed. The h-gate, responsible for inactivation of the sodium ion channel, has a probability of being open of one. Both h and m gates must be open to allow sodium ions into the neuron, facilitating an action potential. The lower graph shows that the time constant of h-gate transitions is substantially greater than that of m-gate transitions at all transmembrane potentials and especially at the transmembrane resting potential. Thus, a preferred embodiment of the present invention allows for a gate transition from h-open to h-closed while disallowing the m-gates in an axon to open with sufficient probability to generate an action potential. This is facilitated by the transition from anodic hyperpolarization stimulation to kHz AC stimulation.

Advantages achieved by the invention include:

1) an implantable apparatus utilizing a cervical multi-contact nerve cuff electrode capable of selectively stimulating the vagus nerve for cardiovascular effects via a multi-phase waveform;
2) the stimulation method maximizes the ratio of heart rate reduction to side effects caused by unwanted stimulation of the larynx and pharynx; and
3) the stimulation method limits the recruitment of large-diameter fibers, associated with side effects, to the first pulse of a therapy pulse.

Although an exemplary embodiment of the present invention has been shown and described, it should be apparent to those of ordinary skill that a number of changes and modifications to the invention may be made without departing from the scope of the invention. In particular, it is possible to implant the apparatus on either vagus nerves. This invention can readily be adapted to a number of different kinds of implantable pulse generators and nerve stimulators by following the present teachings.

**List of reference signs**

| | |
|---|---|
| 20 | IPG case |
| 22 | header |
| 24; 26; 28; 30; 32; 34 | connectors |
| 36; 38; 40; 42; 44; 46 | stimulation units |
| 50 | control unit |
| 52 | time base generator |
| 54 | activity sensing unit |
| 56 | far-field electrogram sensing unit |
| 60 | impedance measurement unit |
| 62 | current source |
| 64 | voltage sensing unit |
| 66 | impedance determination unit |
| 70 | memory unit |
| 72 | telemetry unit |
| 100 | nerve cuff electrode |
| 102; 102.1; 102.2 | contacts |
| 103 | right vagus nerve |
| 104, 104.1 | ring electrode(s) |
| 104.2 | second ring electrode |
| 105 | biocompatible strings |
| 106 | implantable pulse generator (IPG) |
| 107 | implantable electric conduit |
| 200 | electrical pulse |
| 201 | quasi-trapezoidal envelope |
| 300 | non-pure sinusoidal waveform |
| 301 | amplitude |
| 302 | pseudo pulses |
| 303 | stimulation period |
| 400 | switched Class-E amplifier |
| 401 | inductance L |
| 402 | capacitor C |
| 403; 403.1; 403.2;403.3; 403.4 | DC blocking capacitors |
| 404 | electrodes |
| 405 | electrode-tissue impedance |
| 406 | analog switch |
| 407 | $V_{drive}$ |
| 408 | node |
| 409 | $V_{supply}$ |
| 411 | capacitance C |
| 500.1; 500.2; 500.3; 500.4 | analog switches |
| 501 | circuit ground |
| 502.1; 502.2 | analog switches |
| 503.1 | parasitic diode |
| 504.1; 504.2; 504.3; 504.4 | electrode-tissue impedance |
| 505 | resistor |
| 506 | analog switch |
| 800 | charge-balanced alternating current (AC) waveform |
| 900 | selective-arrest phase |
| 901 | transition to steady state |
| 902 | passive charge-balancing phase |
| 903 | therapy pulse |
| 904 | global balancing phase |
| 1200.1; 1200.2 | PMOS transistors |
| 1201 | NMOS transistor |

| 1202 | gate |
| 1203 | resistor |
| 1300.1; 1300.2 | NMOS transistors |
| 1301 | PMOS transistor |
| 1302 | source |
| 1303 | gate |
| 1304 | resistor |
| 1305 | gate |
| 1400.1; 1400.2; 1400.3; 1400.4 | analog switches |
| 1401 | stimulating current |
| 1402.1; 1402.2 | electrodes |
| 1403 | analog switch |
| 1404 | current-limiting resistor |
| 1500 | selective-arrest phase |
| 1501 | charge-balanced rectangular waveform |
| 1502 | square pulses |
| 1503 | passive-charge balancing |
| 1504 | passive-charge balancing |

**Claims**

1. Implantable pulse generator (106) that is connected or can be connected to a stimulation lead (100) having a plurality of stimulation electrodes (102, 104) for delivery of stimulation pulses

   said implantable pulse generator (106) comprising at least one stimulation unit(36, 38, 40, 42, 44, 46, 48) that is configured to generate electric stimulation pulses for nerve stimulation,

   said implantable pulse generator (106) further comprising a control unit (50) that is configured to trigger delivering of generated electric stimulation pulses via selected electrodes of the plurality of stimulation electrodes (102, 104) wherein said electric stimulation pulses form a train comprising:

   i) an initial selective-arrest phase for the large-diameter fibers in the vicinity of the selected electrodes;
   ii) followed by a phase where charge-balanced Alternating Current (AC) is applied between the same or other selected electrodes;
   iii) and where such AC is briefly unbalanced to effectively deliver nerve stimulation therapy pulses, returning to charge-balanced operation in between therapy pulses.

2. Implantable pulse generator (106) according to claim 1, wherein said plurality of stimulation electrodes (102, 104) comprises at least one ring electrode (104) and a plurality of contacts (102) that are electrodes with a smaller contact surface than said ring electrode (104).

3. Implantable pulse generator (106) according to claim 2, wherein said plurality of stimulation electrodes (102, 104) comprises at least two ring electrodes (104.1, 104.2) that are axially spaced from each other and wherein said contacts (102) are arranged between said at least two ring electrodes (104.1, 104.2).

4. Implantable pulse generator (106) according to at least one of claims 1 to 3, wherein said stimulation lead (100) is a multi-contact nerve cuff electrode (100).

5. Implantable pulse generator (106) according to at least one of claims 1 to 4, wherein said control unit (50) is configured to trigger a pulse train for nerve stimulation therapy that is time duty-cycled.

6. Implantable pulse generator (106) according to at least one of claims 1 to 5, wherein said control unit (50) is configured to trigger bipolar stimulation between two of the contacts (102), or a contact (102) and at least one ring (104).

7. Implantable pulse generator (106) according to at least one of claims 1 to 6, wherein said control unit (50) is configured to terminate nerve stimulation therapy by interruption of the pulse train.

8. Implantable pulse generator (106) according to at least one of claims 1 to 7, wherein said control unit (50) is configured to trigger delivery of a pulse train effecting a passive charge-balancing phase involving short circuiting of the selected active contacts) (102) and ring(s) (104) as required during the pulse train and immediately after termination of nerve stimulation therapy.

9. Implantable pulse generator (106) according to claim 8, wherein said charge-balancing period involves two stages with different contact(s) (102) and ring(s) (104).

10. Implantable pulse generator (106) according to at least one of claims 1 to 9, wherein said at least one stimulation unit (36, 38, 40, 42, 44) is configured to generate alternating current (AC).

11. Implantable pulse generator (106) according to claim 10, wherein said at least one stimulation unit (36, 38, 40, 42, 44) comprises or is connected to a switched amplifier for generating said alternating current (AC).

12. Implantable pulse generator (106) according to claim 11, wherein said switched amplifier is a low-Q Class-E amplifier that generates a high frequency alternating current (AC) with a main frequency from hundreds of Hz to tens of kHz.

13. Implantable pulse generator (106) according to at least one of claims 1 to 12, wherein said implantable pulse generator (106) is configured to effect said selective-arrest phase by rectification of the alternating current (AC) with a suitable envelope, in particular an envelope approximating a quasi-trapezoidal (QT) pulse.

14. Implantable pulse generator (106) according to at least one of claims 1 to 13, wherein said implantable pulse generator (106) is configured to effect said selective-arrest phase by means of pre-depolarization sub-threshold pulses.

**Patentansprüche**

1. Implantierbarer Impulsgenerator (106), der mit einem Stimulationsdraht (100) verbunden oder verbindbar ist, der mehrere Stimulationselektroden (102, 104) für die Abgabe von Stimulationsimpulsen aufweist, wobei der implantierbare Impulsgenerator (106) mindestens eine Stimulationseinheit (36, 38, 40, 42, 44, 46, 48) umfasst, die dafür ausgelegt ist, elektrische Stimulationsimpulse für eine Nervenstimulation zu erzeugen, wobei der implantierbare Impulsgenerator (106) ferner eine Steuereinheit (50) umfasst, die dafür ausgelegt ist, die Abgabe erzeugter elektrischer Stimulationsimpulse über ausgewählte Elektroden von den mehreren Stimulationselektroden (102, 104) auszulösen, wobei die elektrischen Stimulationsimpulse eine Folge bilden, umfassend:

    i) eine anfängliche Phase eines selektiven Arrests für die Fasern in der Nähe der ausgewählten Elektroden, die einen großen Durchmesser aufweisen;
    ii) gefolgt von einer Phase, wo ein ladungsausgeglichener Wechselstrom (AC) zwischen denselben oder anderen ausgewählten Elektroden angelegt wird;
    iii) und wobei dieser Wechselstrom kurz unausgeglichen gemacht ist, um auf effektive Weise Nervenstimulationstherapieimpulse abzugeben, wobei zwischen Therapieimpulsen zum ladungsausgeglichenen Betrieb zurückgekehrt wird.

2. Implantierbarer Impulsgenerator (106) nach Anspruch 1, wobei die mehreren Stimulationselektroden (102, 104) mindestens eine Ringelektrode (104) und mehrere Kontakte (102) umfassen, wobei es sich bei Letzteren um Elektroden mit einer kleineren Kontaktfläche als die Ringelektrode (104) handelt.

3. Implantierbarer Impulsgenerator (106) nach Anspruch 2, wobei die mehreren Stimulationselektroden (102, 104) mindestens zwei Ringelektroden (104.1, 104.2) umfassen, die axial voneinander beabstandet sind, und wobei die Kontakte (102) zwischen den mindestens zwei Ringelektroden (104.1, 104.2) angeordnet sind.

4. Implantierbarer Impulsgenerator (106) nach mindestens einem von Anspruch 1 bis 3, wobei der Stimulationsdraht (100) eine Nerven-Cuff-Elektrode (100) mit mehreren Kontakten ist.

5. Implantierbarer Impulsgenerator (106) nach mindestens einem von Anspruch 1 bis 4, wobei die Steuereinheit (50) dafür ausgelegt ist, eine Impulsfolge für eine Nervenstimulationstherapie auszulösen, die auf einem Tastverhältnis basiert.

6. Implantierbarer Impulsgenerator (106) nach mindestens einem von Anspruch 1 bis 5, wobei die Steuereinheit (50) dafür ausgelegt ist, eine bipolare Stimulation zwischen zwei von den Kontakten (102) oder einem Kontakt (102) und mindestens einem Ring (104) auszulösen.

7. Implantierbarer Impulsgenerator (106) nach mindestens einem von Anspruch 1 bis 6, wobei die Steuereinheit (50) dafür ausgelegt ist, die Nervenstimulationstherapie durch Unterbrechen der Impulsfolge zu beenden.

8. Implantierbarer Impulsgenerator (106) nach mindestens einem von Anspruch 1 bis 7, wobei die Steuereinheit (50) dafür ausgelegt ist, nach Bedarf während der Impulsfolge und unmittelbar nach der Beendigung der Nervenstimulationstherapie die Abgabe einer Impulsfolge auszulösen, die eine passive ladungsausgleichende Phase bewirkt, die ein Kurzschließen des (mindestens einen) ausgewählten Kontakts (102) und des (mindestens einen) Ringes (104) beinhaltet.

9. Implantierbarer Impulsgenerator (106) nach Anspruch 8, wobei die ladungsausgleichende Periode zwei Stufen mit (mehreren) unterschiedlichen Kontakten (102) und Ringen (104) beinhaltet.

10. Implantierbarer Impulsgenerator (106) nach mindestens einem von Anspruch 1 bis 9, wobei die mindestens eine Stimulationseinheit (36, 38, 40, 42, 44) dafür ausgelegt ist, einen Wechselstrom (AC) zu erzeugen.

11. Implantierbarer Impulsgenerator (106) nach Anspruch 10, wobei die mindestens eine Stimulationseinheit (36, 38, 40, 42, 44) mit einem Schaltverstärker zum Erzeugen des Wechselstroms (AC) ausgestattet oder verbunden ist.

12. Implantierbarer Impulsgenerator (106) nach Anspruch 11, wobei der Schaltverstärker ein Low-Q-Verstärker der Klasse E ist, der einen hochfrequenten Wechselstrom (AC) mit einer Hauptfrequenz von hunderten Hz bis mehreren Zehn kHz erzeugt.

13. Implantierbarer Impulsgenerator (106) nach mindestens einem von Anspruch 1 bis 12, wobei der implantierbare Impulsgenerator (106) dafür ausgelegt ist, die Phase des selektiven Arrestes durch Gleichrichten des Wechselstroms (AC) mit einer geeigneten Enveloppe zu bewirken, insbesondere einer Enveloppe, die einem quasitrapezförmigen Impuls (QT) nahekommt.

14. Implantierbarer Impulsgenerator (106) nach mindestens einem von Anspruch 1 bis 13, wobei der implantierbare Impulsgenerator (106) dafür ausgelegt ist, die Phase des selektiven Arrestes durch Depolarisieren von unterschwelligen Impulsen zu bewirken.

**Revendications**

1. Générateur d'impulsions implantable (106), qui est connecté, ou peut être connecté, à un conducteur de stimulation (100) ayant une pluralité d'électrodes de stimulation (102, 104) pour la délivrance d'impulsions de stimulation, ledit générateur d'impulsions implantable (106) comprenant au moins une unité de stimulation (36, 38, 40, 42, 44, 46, 48) qui est conçue pour générer des impulsions de stimulation électriques pour une stimulation nerveuse, ledit générateur d'impulsions implantable (106) comprenant en outre une unité de commande (50) qui est conçue pour déclencher la délivrance d'impulsions de stimulation électriques générées par le biais d'électrodes choisies dans la pluralité d'électrodes de stimulation (102, 104) où lesdites impulsions de stimulation forment un train comprenant :

i) une phase d'arrêt sélectif initiale pour des fibres de grand diamètre au voisinage des électrodes choisies ;
ii) suivie d'une phase où un courant alternatif (CA) à charge équilibrée est appliqué entre ces électrodes ou d'autres électrodes choisies ;
iii) et où un tel CA est brièvement déséquilibré pour délivrer de manière efficace des impulsions de thérapie de stimulation nerveuse, le retour à un fonctionnement à charge équilibrée entre des impulsions de thérapie.

2. Générateur d'impulsions implantable (106) selon la revendication 1, dans lequel ladite pluralité d'électrodes de stimulation (102, 104) comprend au moins une électrode annulaire (104) et une pluralité de contacts (102) qui sont des électrodes avec une surface de contact plus petite que ladite électrode annulaire (104).

3. Générateur d'impulsions implantable (106) selon la revendication 2, dans lequel ladite pluralité d'électrodes de stimulation (102, 104) comprend au moins deux électrodes annulaires (104.1, 104.2) qui sont espacées axialement l'une de l'autre et dans lequel lesdits contacts (102) sont agencés entre lesdites au moins deux électrodes annulaires (104.1, 104.2).

4. Générateur d'impulsions implantable (106) selon au moins l'une des revendications 1 à 3, dans lequel ledit conducteur de stimulation (100) est une électrode à manchette multi-contact pour un nerf (100).

5. Générateur d'impulsions implantable (106) selon au moins l'une des revendications 1 à 4, dans lequel ladite unité de commande (50) est conçue pour déclencher un train d'impulsions pour une thérapie de stimulation nerveuse qui est à rapport cyclique avec le temps.

6. Générateur d'impulsions implantable (106) selon au moins l'une des revendications 1 à 5, dans lequel ladite unité de commande (50) est conçue pour déclencher une stimulation bipolaire entre deux des contacts (102), ou un contact (102) et au moins un anneau (104).

7. Générateur d'impulsions implantable (106) selon au moins l'une des revendications 1 à 6, dans lequel ladite unité de commande (50) est conçue pour terminer une thérapie de stimulation nerveuse par une interruption du train d'impulsions.

8. Générateur d'impulsions implantable (106) selon au moins l'une des revendications 1 à 7, dans lequel ladite unité de commande (50) est conçue pour déclencher la délivrance d'un train d'impulsions produisant un effet de phase d'équilibrage de charge passive impliquant un court-circuitage du(des) contact(s) actif(s) choisi(s) (102) et de(des) l'anneau(anneaux) (104) comme requis durant le train d'impulsions et immédiatement après la fin de la thérapie de stimulation nerveuse.

9. Générateur d'impulsions implantable (106) selon la revendication 8, dans lequel ladite période d'équilibrage de charge implique deux stades avec un(des) contact(s) (102) et un(des) anneau(x) (104) différent(s).

10. Générateur d'impulsions implantable (106) selon au moins l'une des revendications 1 à 9, dans lequel ladite au moins une unité de stimulation (36, 38, 40, 42, 44) est conçue pour générer du courant alternatif (CA).

11. Générateur d'impulsions implantable (106) selon la revendication 10, dans lequel ladite au moins une unité de stimulation (36, 38, 40, 42, 44) comprend, ou est connectée à, un amplificateur commuté pour générer ledit courant alternatif (CA).

12. Générateur d'impulsions implantable (106) selon la revendication 11, dans lequel ledit amplificateur commuté est un amplificateur de classe E de facteur Q faible qui génère un courant alternatif (CA) de fréquence élevée avec une fréquence principale allant de centaines de Hz à des dizaines de kHz.

13. Générateur d'impulsions implantable (106) selon au moins l'une des revendications 1 à 12, où ledit générateur d'impulsions implantable (106) est conçu pour avoir un effet sur ladite phase d'arrêt sélectif par une rectification du courant alternatif (CA) avec une enveloppe appropriée, en particulier, une enveloppe approchant d'une impulsion quasi trapézoïdale (QT).

14. Générateur d'impulsions implantable (106) selon au moins l'une des revendications 1 à 13, où ledit générateur d'impulsions implantable (106) est conçu pour avoir un effet sur ladite phase d'arrêt sélectif au moyen d'impulsions infraliminaires de pré-dépolarisation.

FIG. 1

EP 3 085 414 B1

Fig. 2

**FIG. 3**

**FIG. 4**

I(405)

410

1.8mA
1.6mA
1.4mA
1.2mA
1.0mA
0.8mA
0.6mA
0.4mA
0.2mA
0.0mA
1.8mA
1.8mA
1.8mA

405

5.0ms 5.1ms 5.2ms

400

401
408 403
402
406
404.1
405 Z
404.2
$V_{supply}$ 409
$V_{drive}$ 407

**FIG. 5**

FIG. 6

FIG. 7

EP 3 085 414 B1

Fig. 8

Fig. 9

FIG. 10

EP 3 085 414 B1

FIG. 11

FIG. 12

FIG. 13

FIG. 14

FIG. 15

EP 3 085 414 B1

Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5199430 A
- US 7389145 B2
- US 20100191311 A1
- US 8615294 B2

**Non-patent literature cited in the description**

- **BARATTA et al.** Orderly Stimulation of Skeletal Muscle Motor Units with Tripolar Nerve Cuff Electrode. *IEEE Transactions on Biomedical Engineering,* August 1989, vol. 39 (8), 836-843